# EUROPEAN PATENT SPECIFICATION

(11) **EP 1 214 077 B1**
(45) Date of publication and mention of the grant of the patent: **28.01.2004**
(21) Application number: 00963838.8
(22) Date of filing: 21.09.2000
(51) Int. Cl.: A61K 31/565, A61P 41/00

(54) **LOCAL DELIVERY OF 17-BETA ESTRADIOL FOR IMPROVING VASCULAR ENDOTHELIUM FUNCTION AFTER VASCULAR INJURY**
LOKALE VERABREICHUNG VON 17-BETA ESTRADIOL ZUR VERBESSERUNG DER VASKULAREN ENDOTHELIALEN FUNKTION NACH GEFÄSSVERLETZUNGEN
ADMINISTRATION LOCALE DE 17-BETA ESTRADIOL POUR LE RENFORCEMENT DES FONCTIONS DE L'ENDOTHELIUM VASCULAIRE APRES UNE LESION VASCULAIRE

(30) Priority: 21.09.1999 CA 2282982; 09.03.2000 CA 2300246
(43) Date of publication of application: 19.06.2002
(73) Proprietor: Institut de Cardiologie de Montreal, Montreal, Quebec H1T 1C8 (CA)
(72) Inventor: CHANDRASEKAR, Baskaran, Chennai 600 041 (IN); TANGUAY, Jean-François, Monreal, Quebec H3P 3C5 (CA)
(74) Representative: Vercaemer, Laurence
(86) International application number: PCT/CA2000/001132
(87) International publication number: WO 2001/021157

(56) References cited:
- WO-A-00/10552
- WO-A-00/12147
- WO-A-98/56312
- US-A- 5 376 652
- US-A- 5 866 561
- YOON YOUNG-SUP ET AL: "Local intraluminal infusion of oestradiol containing liposome in rat cartoid balloon injury model." EUROPEAN HEART JOURNAL, vol. 19, no. ABST. SUPPL., August 1998 (1998-08), page 616 XP001014473 XXth Congress of the European Society of Cardiology;Vienna, Austria; August 22-26, 1998 ISSN: 0195-668X
- ZHANG Y-Q ET AL: "EFFECTS OF GENDER AND ESTRADIOL TREATMENT ON FOCAL BRAIN ISCHEMIA" BRAIN RESEARCH, AMSTERDAM, NL, vol. 784, no. 1/2, 16 February 1998 (1998-02-16), pages 321-324, XP000884355 ISSN: 0006-8993
- KAUFFMAN RAYMOND F ET AL: "Comparative effects of local vs. systemic administration of estrogen upon vascular responses to balloon injury." CIRCULATION, vol. 92, no. 8 SUPPL., 1995, page I628 XP001014766 68th Scientific Session of the American Heart Association;Anaheim, California, USA; November 13-16, 1995 ISSN: 0009-7322
- DATABASE BIOSIS [Online] BIOSCIENCES INFORMATION SERVICE, PHILADELPHIA, PA, US; May 1999 (1999-05) PERROT-APPLANAT M: "Qestradiol and proliferation of vascular cells." Database accession no. PREV200000507148 XP002177140 & THERAPIE (LONDON), vol. 54, no. 3, May 1999 (1999-05), pages 333-337, ISSN: 0040-5957
- DATABASE BIOSIS [Online] BIOSCIENCES INFORMATION SERVICE, PHILADELPHIA, PA, US; 1995 MIKKOLA TOMI ET AL: "17-beta-estradiol stimulates prostacyclin, but not endothelin-1, production in human vascular endothelial cells." Database accession no. PREV199598355996 XP002177141 & JOURNAL OF CLINICAL ENDOCRINOLOGY & METABOLISM, vol. 80, no. 6, 1995, pages 1832-1836, ISSN: 0021-972X
- DATABASE BIOSIS [Online] BIOSCIENCES INFORMATION SERVICE, PHILADELPHIA, PA, US; 1996 MIKKOLA TOMI ET AL: "Effect of physiological concentrations of estradiol on PGI-2 and NO in endothelial cells." Database accession no. PREV199799286811 XP002177142 & MATURITAS, vol. 25, no. 2, 1996, pages 141-147, ISSN: 0378-5122
- CHANDRASEKAR, BASKARAN (1) ET AL: "Coronary artery endothelial protection following local delivery of 17-beta estradiol during balloon angioplasty: A potential new pharmacological approach to improve long-term outcome of angioplasty." JOURNAL OF THE AMERICAN COLLEGE OF CARDIOLOGY., (FEB., 2000) VOL. 35, NO. 2 SUPPL. A, PP. 58A. MEETING INFO.: 29TH ANNUAL SCIENTIFIC SESSION OF THE AMERICAN COLLEGE OF CARDIOLOGY. ANAHEIM, CALIFORNIA, USA MARCH 12-15, 2000 , XP001015229
- SELZMAN, CRAIG H. ET AL: "Estrogen replacement inhibits intimal hyperplasia and the accumulation and effects of transforming growth factor.beta.1" J. SURG. RES. (1998), 80(2), 380-385 , XP001014759
- KEANEY J F ET AL: "17BETA-ESTRADIOL PRESERVES ENDOTHELIAL VASODILATOR FUNCTION AND LIMITS LOW-DENSITY LIPOPROTEIN OXIDATION IN HYPERCHOLESTEROLEMIC SWINE" CIRCULATION, AMERICAN HEART ASSOCIATION, DALLAS, TX, US, vol. 89, no. 5, May 1994 (1994-05), pages 2251-2259, XP001014735 ISSN: 0009-7322
- DATABASE BIOSIS [Online] BIOSCIENCES INFORMATION SERVICE, PHILADELPHIA, PA, US; 1996 KUBLIK HEIKE ET AL: "Nasal absorption of 17-beta-estradiol from different cyclodextrin inclusion formulations in sheep." Database accession no. PREV199799383935 XP002177143 & EUROPEAN JOURNAL OF PHARMACEUTICS AND BIOPHARMACEUTICS, vol. 42, no. 6, 1996, pages 320-324, ISSN: 0939-6411

## Description

### FIELD OF THE INVENTION

The present invention relates to the local use of estradiol or a derivative thereof for the manufacture of a medicament or device intended to improve the outcome of a coronary angioplasty. More specifically, the present invention is concerned with the local use of estradiol or a derivative thereof the manufacture of a medicament or device for improving the endothelium function after vascular injury, both events contributing to the ultimate success of an angioplasty.

### BACKGROUND OF THE INVENTION

Restenosis is currently the major limitation of percutaneous transluminal coronary angioplasty (PTCA), and is seen in up to 30-40 % of patients.¹ The most important mechanisms contributing to restenosis are neointima proliferation, vascular remodelling, and elastic recoil.² Elastic recoil and vascular remodelling can be reduced to a large extent by stenting.³ Although radiation therapy has been reported to show beneficial effects,^{4,5} no effective therapy exists yet for neointima proliferation. Vascular smooth muscle cell (SMC) migration and proliferation have been documented to occur as early as 36 hours following arterial injury.⁶ In cell culture assays, 17-beta estradiol inhibited migration and proliferation of rat vascular SMC.^{7,8} Similar effects have also been shown with human vascular SMC from saphenous vein.⁹ Prolonged systemic administration of estrogen has been shown to inhibit intima hyperplasia in animal studies.^{10,11} Instead of administrating estradiol systematically we here tested how a local administration of 17-beta estradiol during PTCA could effectively inhibit neointima proliferation.

The vital role of endothelium in the regulation of vascular tone of arteries is well-recognized (1). The intact endothelium also has important inhibitory effects on platelet aggregation, monocyte adhesion, and vascular smooth muscle cell proliferation (2). Endothelial injury associated with endothelial dysfunction is known to occur as a consequence of percutaneous transluminal coronary angioplasty (PTCA) (3), and may play an important role in restenosis following PTCA (4). Impaired endothelial function has been demonstrated in porcine coronary arteries as long as 4 weeks following PTCA in pigs (5). Systemically administered 17-beta estradiol has been reported to accelerate endothelial recovery after arterial injury (10). Since endothelial injury due to PTCA is a local event, we hypothesized that local delivery of 17-beta estradiol following PTCA may enhance endothelial recovery.

### SUMMARY OF THE INVENTION

An object of the present invention is therefore to provide efficient uses of 17-β estradiol or a derivative thereof for the manufacture of a medicament or device which is intended to be used locally during PTCA to improve endothelial function after vascular injury. Compositions for executing these uses are also a further object of this invention.

Other objects, advantages and features of the present invention will become more apparent upon reading of the following nonrestrictive description of preferred embodiments thereof, given by way of examples only, with reference to the accompanying drawings.

### BRIEF DESCRIPTION OF THE DRAWINGS

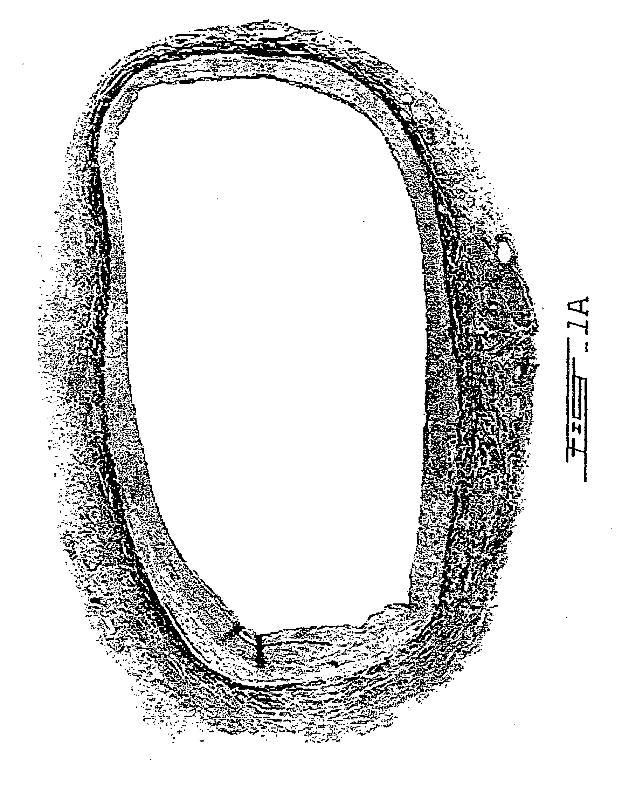

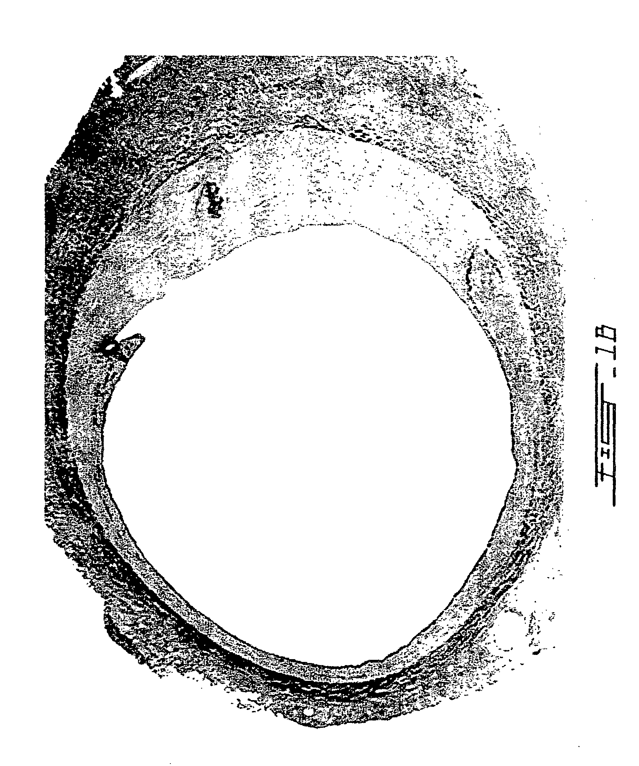

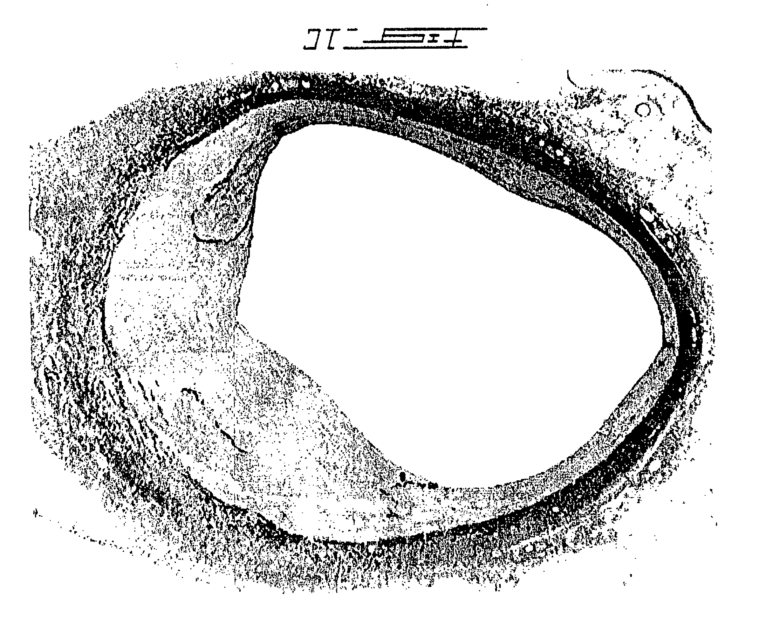

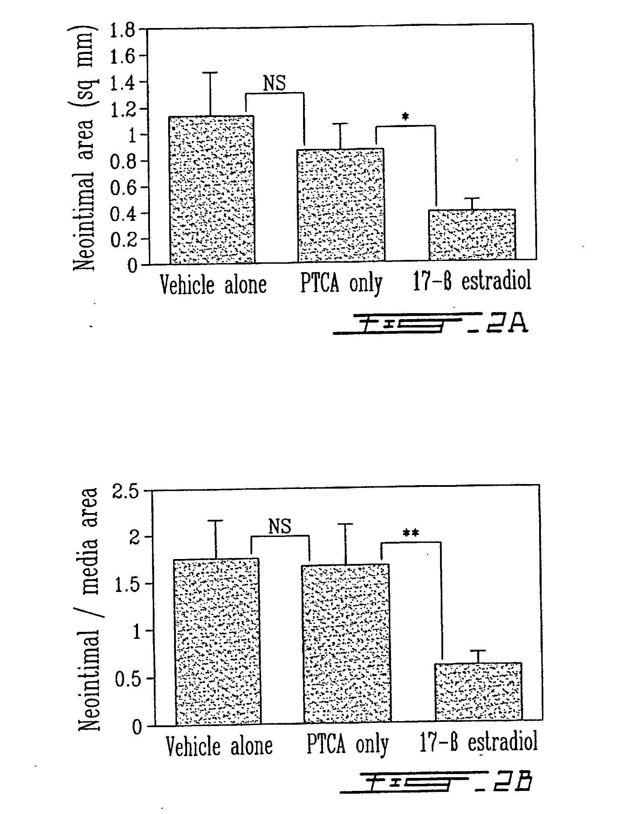

**Figure 1** Representative light micrographs (x 40 magnification) of arterial segments from the same animal, stained with Verhoeffs stain. 17-beta estradiol (a) treated segment shows markedly less neointima hyperplasia compared to PTCA only (b), or vehicle alone (c) groups. The extent of injury is similar in all 3 segments.

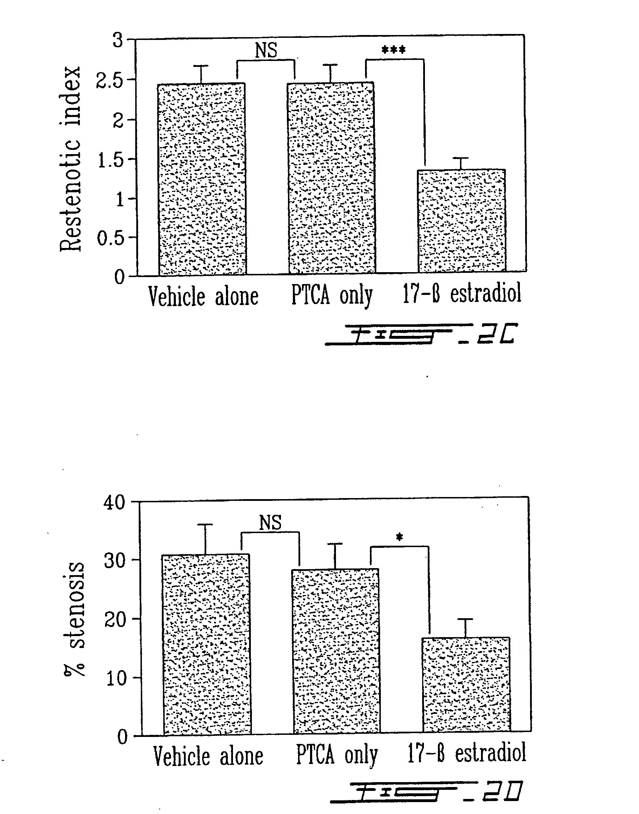

**Figure 2** Comparison of (A) neointima area, (B) neointima/media area, (C) restenotic index, and (D) % stenosis between PTCA alone vs vehicle only, and PTCA only vs 17-beta estradiol groups; * p < 0.05, ** p < 0,01 *** p < 0.002. Values are expressed as mean ± SEM.

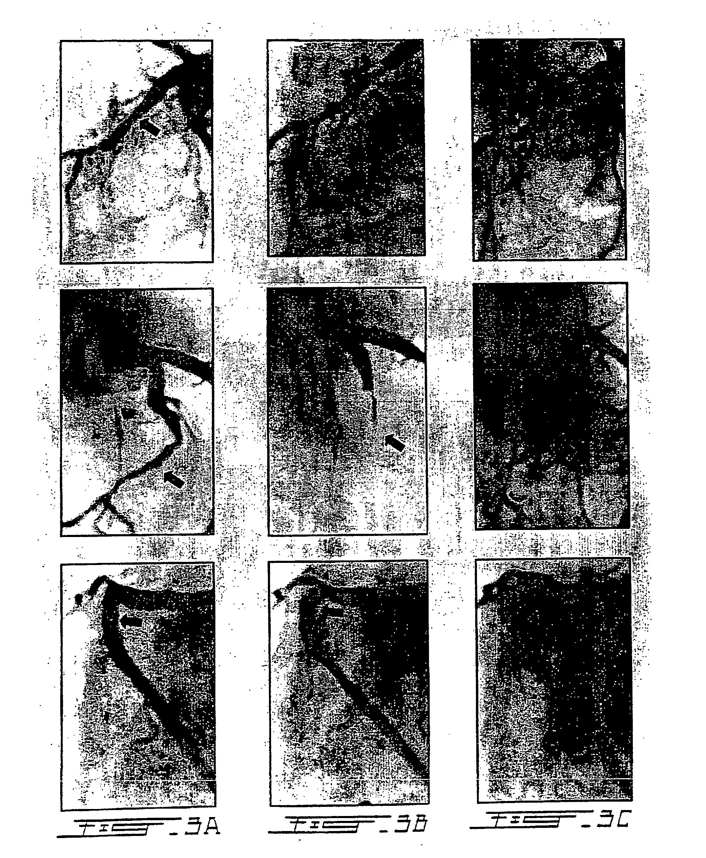

**Figure 3** Representative coronary angiograms demonstrating the vasoconstrictive response to intracoronary infusion of acetylcholine (Ach) 10⁻⁴M obtained from the same animal at 4 weeks following percutaneous transluminal coronary angioplasty (PTCA). Column A = basal, column B = after Ach, column C = following intracoronary nitroglycerin. Top panel = treatment with vehicle, mid panel = PTCA only, lower panel 17-beta estradiol treatment groups respectively.

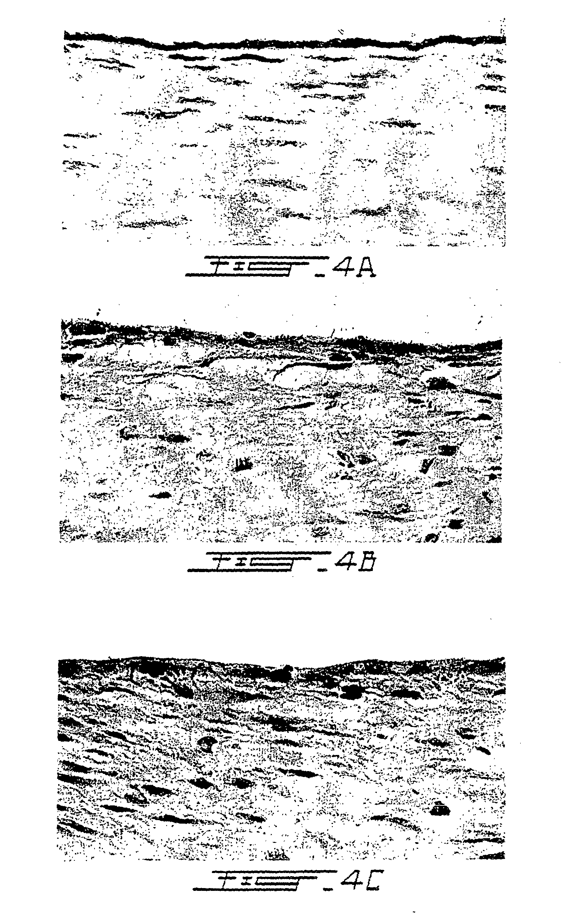

**Figure 4** Representative light micrographs (x 1000) of cross sections of vessels obtained from the same animal for immunohistochemical staining with the lectin *Dolichos biflorus* agglutinin (evident as dark brown staining of luminal surface). Vessels treated with 17-beta estradiol (A) demonstrate reendothelialization to a greater degree as compared to PTCA only (B) and vehicle (C) groups.

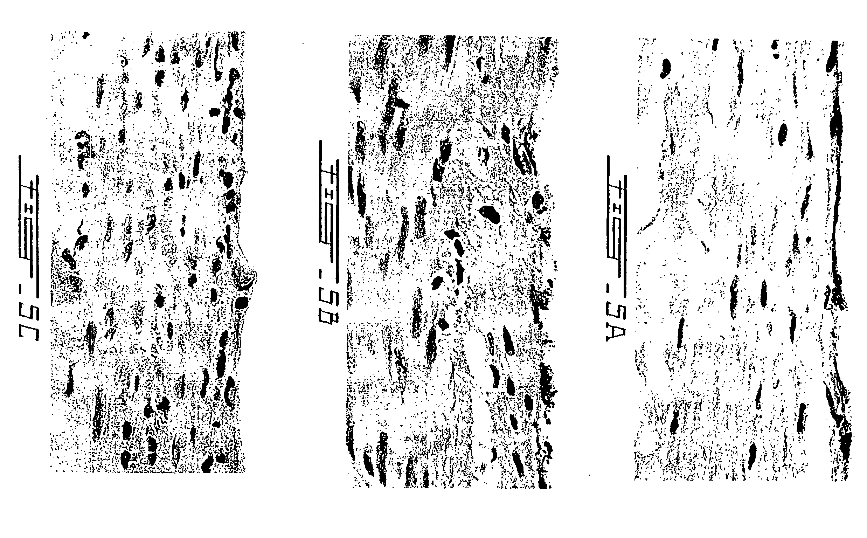

**Figure 5** Representative light micrographs (x 1000) of cross sections of vessels obtained from the same animal, for immunohistochemical analysis of endothelial nitric oxide synthase (eNOS) expression. Vessels treated with 17-beta estradiol (A) show greater expression of eNOS (evident as dark brown staining of luminal surface) as compared to PTCA only (B) and vehicle (C) groups.

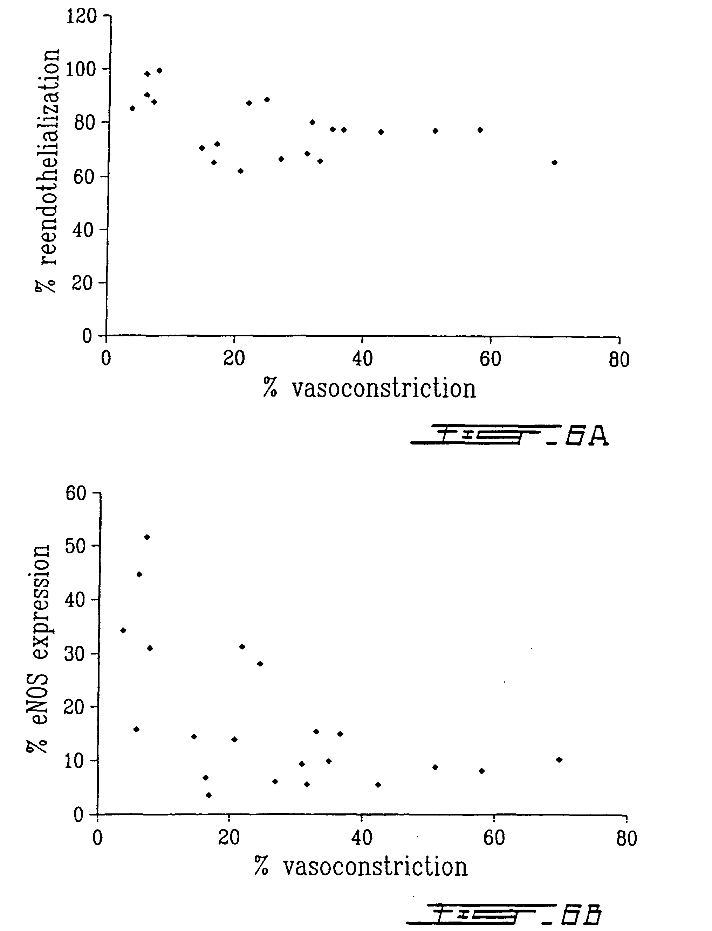

**Figure 6** Graph depicting correlation between vasoconstrictive response to Ach 10⁻⁴ M and (A) reendothelialization (r = -0.48, p < 0.02), (B) eNOS expression (r = -0.58, p < 0.005). Note: % vasoconstriction denotes % decrease in diameter following Ach 10⁻⁴ M as compared to the basal diameter.

### DESCRIPTION OF THE PREFERRED EMBODIMENTS

### Example 1: The effect of estradiol on neointima hyperplasia

### Methods

### Animal preparation

Eighteen juvenile farm pigs (9 female, and 9 castrated male) weighing 20-25 kg were studied. The study was approved by, and conducted in accordance with, the guidelines of the Animal Care and Ethical Research Committee of the Montreal Heart Institute. Before the procedure, animals were given 650 mg of acetylsalicylic acid and 30 mg of nifedipine orally, premedicated with intramuscular injection of 6 mg/kg of a mixture of tiletamine hydrochloride and zolazepam hydrochloride, and given 0.05 mg of atropine. The invasive procedure was performed under general anesthesia with a mixture of isoflurane (1 to 1.5 %) and oxygen enriched air. The right femoral artery was cannulated percutaneously, and an 8 Fr arterial sheath was introduced. After arterial access had been obtained, 100 mg of lidocaine and 250 U/kg of heparin were administered intra-arterially via the sheath. Activated coagulation time was maintained at > 300 seconds throughout the procedure.

### Preparation of estradiol formulation

Each dose individually administered to the tested animals is composed of at least 12.5 mg hydroxypropyl-beta-cyclodextrin (HPCD) and 600 µg estradiol in a 5 ml solution volume.

A smaller or larger dose may be used. Indeed, the tested dose corresponds to the dose of about 675 µg formulated in a sublingual pellet and administered to postmenopausal women.⁴⁵ Such a dose may be unnecessarily high if administered locally. Indeed, doses of 200 and 400 µg have been tried and they were found to be as performing as the dose of 600 µg. Further, the necessary dose for performing the present invention may be influenced by the hormonal balance of the individual to be treated. Species variance is also a factor affecting the dosage regimen. Also, any derivative of 17-beta estradiol may replace the latter. A derivative is intended to cover a precursor, an active metabolite, an active analog or a modulator capable of positively influencing the activity of the receptor(s) to estradiol or of enhancing the binding and/or the activity of estradiol towards its receptor(s). Such derivatives are considered functional equivalents of 17-beta-estradiol, and therefore within the scope of this invention. A unit dose of 1 to 5000 µg/Kg of 17-beta-estradiol or an equivalent derivative dose is within the scope of this invention, preferably 10-50 µg/Kg, even more preferably 10-30 µg/Kg.

### Angioplasty and Local Delivery

Standard PTCA equipment was used. An 8 Fr right Amplatz guiding catheter and right Judkins guiding catheter were used for cannulation of the left and right coronary arteries, respectively. PTCA was performed with a balloon size chosen to correspond to a balloon/artery ratio of 1.1-1.3. Three 30-second inflations at 10 atm pressure were performed with a 30-second interval between each inflation. Inflations were performed adjacent to major side branches to facilitate identification during harvesting, taking precaution not to include any side branch in the intended PTCA site. The left anterior descending, left circumflex, and right coronary arteries of each animal were subjected to PTCA. After PTCA, each coronary artery of an animal was randomized to receive either 600 µg of 17-beta estradiol locally, or vehicle alone locally, or PTCA only. The chemicals 17-beta estradiol and its vehicle 2-hydroxypropyl-beta-cyclodextrin (HPCD) were purchased from Sigma Chemical Co. The InfusaSleeve catheter (Local Med, Inc.) was used for local delivery.¹² Five ml of the designated substance was delivered at a driving pressure of 10 atm and support balloon pressure of 6 atm.

Of the 18 animals, 2 died a few days after PTCA, and were excluded; thus, 16 animals were analyzed. Twelve animals were euthanized at 28 days, and 4 at 7 days. After premedication and anesthesia, the right internal jugular vein and common carotid artery were cannulated. Following cross-clamping of the descending thoracic aorta exposed via a left lateral thoracotomy, exsanguination was performed, with simultaneous administration of 1 I of 0.9 % NaCl solution. The heart was perfusion-fixed in vivo with 2 I of 10 % buffered formalin at 200 mm Hg pressure, removed from the animal, and placed in 10 % buffered formalin solution. Coronary arteries were then dissected free from surrounding tissues. The site of PTCA was identified in relation to adjacent side branches, which served as landmarks. The injured segment was harvested with a 1 cm normal segment proximal and distal to the injured site. Serial sections 3 to 5 mm long were made from the harvested segment, with a minimum of at least 3 sections (maximum 5) from each PTCA site. Sections were stored in buffered 10 % formalin and subjected to dehydration with increasing concentrations of alcohol, followed by treatment with xylene and paraffin. Each section was then cut to slices of 6 µm thickness with a microtome (Olympus cut 4060 E), and stained with Verhoeff's stain for morphometric analysis.

### Morphometric analysis

Measurements were made with a video microscope (Leitz Diaplan, equipped with a Sony DXC 970 MD color video camera) linked to a 486 personal computer and customized software. A minimum of 3 sections for each injured segment were analyzed and results averaged. Analyses were made by a single observer unaware of the treatment group to which each segment had bee allocated. Randomly selected sections were viewed by a second observer (also blinded to protocol) independently; inter-observer variability was < 5 %. The areas of external elastic lamina (EEL), internal elastic lamina (IEL), and lumen were measured by digital planimetry; neointima (I) area (IEL - lumen area) and media (M) area (EEL - IEL area) were obtained. The % neointima was defined as the % of total vessel area occupied by neointima (% neointima = [I/EEL] x 100). Morphologic % stenosis was calculated as 100 (1 ― lumen/IEL area).¹³ The restenotic index was defined as [I/(I + M)]/(F/IEL circumference), where F is the fracture length of internal elastic lamina.¹⁴ Histologic injury score was determined as previously defined.¹⁵

### Immunohistochemistry

Following slicing with a microtome and blocking of non-specific antibodies, the sections were treated with mouse anti-proliferating cell nuclear antigen (PCNA) antibodies and diluted biotinylated goat anti-mouse antibodies. They were then incubated with avidin-biotin (Elite ABC Kit, Vector Laboratories), and developed with 3, 3'-diaminobenzidine (Vector Laboratories). They were finally counter-stained with hematoxylin. Porcine liver cells were used as a positive control. For each section, a 6 µm slice counter-stained with hematoxylin without treatment with the primary antibody (mouse anti- PCNA) served as a negative control.

The proliferative response to injury was studied by immunohistochemical analysis of samples from animals euthanized at 7 days. The % proliferating SMC was obtained by dividing the number of PCNA - positive SMC by the total number of SMC in each field; separate measurements were made for neointima and media layers. The proliferating cells were identified as SMC by positive staining of parallel sections with a smooth muscle actin antibody. To standardize comparison among treatment groups, measurements were obtained at 4 fixed locations separated by 90° sites for each section, and the results averaged. For each segment, two sections demonstrating maximal neointima response were analyzed, and the results averaged.

### Statistical Analysis

Values are expressed as mean t standard deviation, except as otherwise indicated. Kruskal - Wallis analysis was used for comparison of data among the 3 groups; subsequently, 17-beta estradiol and vehicle alone groups were separately compared with the PTCA only group using the Mann - Whitney rank sum test. Chi - square analysis was used for comparison of proportions.

The Mann - Whitney rank sum test was also used for comparison of data between male and female animals within the 17-beta estradiol treated group. Values were considered statistically significant if p < 0.05.

### Results

Following PTCA and local delivery, animals were allowed to recover, and gained weight steadily. Two animals died 48 and 72 hours after procedure respectively, and were not included; thus 16 animals were studied. Autopsy of the 2 animals revealed occlusive thrombus at the site of PTCA (in the 17-beta estradiol treated vessel in one pig, and in the vessel treated with PTCA only in the other pig).

### Injured segments

Balloon/artery ratio and artery diameter were not significantly different among the 3 treatment groups (Table 1). Segments with intact IEL in which discernible injury was absent were excluded from analysis (2 from PTCA only group, and 1 from vehicle alone group). Two segments were lost during harvesting and processing (1 of vehicle alone, and 1 of PTCA only group).

### Morphometric analysis

Of the 12 animals that underwent morphometric analysis at 28 days, arterial segments treated with local delivery of 17-beta estradiol showed significantly less neointima hyperplasia (Figure 1). This beneficial effect was noted in all parameters of neointima response to injury that were analyzed (Table 1). Of note, the extent of morphologic injury was similar among the 3 groups, suggesting that the use of the InfusaSleeve catheter was not associated with an enhanced risk of injury.

It was important to exclude an inhibitory effect on intima proliferation due to the vehicle, and, to confirm that the effect noted was in response to treatment with 17-beta estradiol. Analyses comparing segments treated with vehicle alone and PTCA only showed a similar response in terms of the extent of neointima proliferation. On the other hand, significantly less intima hyperplasia was observed in 17-beta estradiol treated segments as compared to segments treated with PTCA only (Figure 2). Compared to PTCA only, or vehicle alone, 17-beta estradiol decreased neointima formation by 54.6 % and 64.9 % respectively.

To exclude the possibility of influence of sex on response to estrogen, the 7 segments obtained from male pigs treated with 17-beta estradiol, and 5 segments obtained from female pigs treated with 17-beta estradiol were analyzed. No statistically significant differences were evident (Table 2).

### Immunohistochemistry

The number of PCNA - positive SMC was low overall; sacrifice at an earlier time might have yielded a higher number. However, a statistically significant decrease in the proliferative response was seen in animals treated with 17-beta estradiol. Among the different groups, the % of PCNA - positive SMC in the neointima were 0.43 ± 0.52 % in 17-beta estradiol, 4.26 ± 2.33 % in PTCA only, and 4.27 ± 2.73 % in vehicle alone groups respectively (p < 0.05 for 17-beta estradiol vs other 2 groups). There were no statistically significant differences in % PCNA - positive SMC in the media among the 3 groups: 0.4 ± 0.3 %, 1.38 ± 1.74 %, and 1.24 ± 1.57 % for 17-beta estradiol, PTCA only, and vehicle alone groups respectively (p = NS).

### Vascular remodeling

To determine the effect on vascular remodeling of the agents used, the EEL area of the injured segment and of the normal vessel proximal to site of PTCA were obtained, and their ratio calculated.¹³ No significant difference among the groups was noted: 1.01 ± 0.16, 1.16 ± 0.28, 1.31 ± 0.37 respectively for 17-beta estradiol, PTCA only, and vehicle alone groups respectively (p = NS).

### Conclusions

The present study demonstrates, for the first time, that locally delivered 17-beta estradiol decreases neointima proliferation following PTCA in pigs. The study also shows that the InfusaSleeve catheter can be used to deliver effectively 17-beta estradiol intramurally in coronary arteries.

Several previous experiments in animals have demonstrated that estrogen administered subcutaneously for up to 3 weeks inhibited the myointima response to arterial injury.^{10,11} Recently, short-term subcutaneous estrogen therapy (6 to 17 days) was also shown to be effective in reducing the injury response in rat carotid artery.¹⁶ Estrogen administered intra-muscularly for at least 3 weeks has also demonstrated the potential to inhibit vascular smooth muscle cell proliferation and neointima hyperplasia in rabbits.¹⁷ However, the efficacy of local delivery of 17-beta estradiol to inhibit intima hyperplasia has not been previously studied.

The biologic effects of estrogen, like other steroid hormones, involve intracellular receptors. The first estrogen receptor (ER) to be discovered was ERα,^{18,19} which was thought to mediate the beneficial effects of estrogen following vascular injury. ERα was also present in coronary arteries obtained from autopsy specimens in both pre and postmenopausal women,²⁰ and in cell cultures of human saphenous vein and internal mammary artery specimens.²¹ Recently, a second estrogen receptor, ERβ, has been identified in animals and humans.^{22,23} The role of ERβ in response to vascular injury was subsequently demonstrated in experiments with ERα deficient mice.²⁴ Normal and ERα deficient mice treated with estrogen, when subjected to arterial injury, showed the same extent of inhibition of neointima proliferation compared to control mice; thereby demonstrating that inhibition of vascular injury response by estrogen is independent of ERα. Although the present experiment was not designed to study the mechanism of action of 17-beta estradiol, evidence exists for multiple potential mechanisms by which 17-beta estradiol can inhibit the vascular response to injury. Of importance may be the effect of 17-beta estradiol on nitric oxide (NO) synthesis. In cell culture studies with human and bovine endothelial cells, treatment with 17-beta estradiol stimulated NO synthase and increased NO production.^{25,26} Postmenopausal women treated with transdermal 17-beta estradiol showed enhanced in vivo NO synthesis.²⁷ NO has demonstrated inhibitory effects on both migration ²⁸ and proliferation ²⁹ of vascular SMC, and decreased neointima formation after PTCA.¹³ Preliminary reports have shown that therapy with 17-beta estradiol decreases intercellular and vascular cell adhesion molecule expression by human coronary SMC.³⁰ Cellular adhesion molecules are expressed by SMC following arterial injury³¹ and their suppression with the use of monoclonal antibodies inhibited intima hyperplasia after arterial injury in rats.³² The regulatory effect of 17-beta estradiol on vascular endothelial growth factor expression may also be partly responsible.³³⁻³⁵ Perhaps the most important mechanism may be a direct inhibitory effect of 17-beta estradiol on vascular SMC proliferation.³⁶ The binding of 17-beta estradiol to its intracellular receptor activates DNA containing "estrogen responsive elements", leading to altered gene expression. 17-beta estradiol also reduces platelet derived growth factor-induced migration and proliferation of vascular SMC.⁹

The beneficial effects of 17-beta estradiol, the predominant circulating estrogen in premenopausal women, on vascular injury response may not be replicated by other kinds of estrogens; for example, conjugated equine estrogen was found to have no effect on neointima proliferation in non-human primate models.³⁷ Simultaneous administration of progesterone may attenuate the vascular injury response to 17-beta estradiol.³⁸ A sexually dimorphic response to estrogen in intact rats has been reported following arterial injury, with male rats deriving no benefit with estrogen therapy .³⁹ This sexually dimorphic effect was, however, not observed in another experiment with gonadectomized rats.¹¹ In the present study, too, no significant difference in neointima proliferative response to 17-beta estradiol was noted between the sexes. Increased expression of ERβ mRNA (ERβ is directly associated with inhibition of vascular SMC proliferation) following arterial injury has been demonstrated in intact male rats;⁴⁰ of additional interest in the study is that no increase in ERα was seen following arterial injury.

17-beta estradiol is a lipophilic compound with poor solubility in aqueous solutions, thereby needing a vehicle for parenteral administration. HPCD is a starch derivative that has been successfully tested as an effective excipient for protein drugs.⁴¹ The pharmacokinetics of HPCD are similar to that of inulin, and the toxic dose (nephrotoxicity) has been estimated to be 200 mg/kg in rats.⁴² The dose of HPCD used to dissolve 17-beta estradiol in the present study was 0.63 mg/kg, far below the toxic dose. Furthermore, HPCD has been used for administration of ophthalmic preparations and intravenous anaesthetic agents in humans.^{43,44} HPCD complexed to 17-beta estradiol has been used to enhance bioavailability of orally, or, sublingually administered 17-beta estradiol with no untoward effects in humans.⁴⁵

Retrospective studies in humans have shown no benefit of hormonal replacement therapy on angiographic restenosis following PTCA⁴⁶ although one study did show a beneficial effect after directional atherectomy.⁴⁷ However, it should be noted that conjugated estrogen (and not 17-beta estradiol) was the predominant form of estrogen used in many of these patients, and, no information about concomitant use of progesterone is available.

In conclusion, we have shown that, a single dose of 17-beta estradiol delivered locally during PTCA has the potential to inhibit neointima proliferation effectively. The delivery of 17-beta estradiol can be performed easily with the InfusaSleeve catheter, without risk of additional injury. With this approach, it may be possible to avoid potential undesirable effects of long term systemic administration of estrogen. ERβ has been identified in humans, and inhibition of proliferation of human vascular SMC by 17-beta estradiol has been demonstrated in cell culture assays. The local administration of 17-beta estradiol is therefore a promising new approach, which might be useful in preventing the proliferative response after PTCA in humans. Its usefulness in preventing restenosis after PTCA is contemplative in view of the foregoing promising results.

### Example 2: The effect of estradiol on vascular endothelial function

### Methods

### Animal preparation

The study protocol was approved by the Animal Care and Ethical Research Committee of the Montreal Heart Institute. Juvenile farm pigs weighing 20-25 kg (1 female, and 8 castrated males) were used. On the day of the experiment, animals received 650 mg of acetylsalicylic acid and 30 mg of nifedipine orally, were premedicated with 6mg/kg of tiletamine hydrochloride and zolazepam hydrochloride, and were given 0.05 mg of atropine intramuscularly. Under general anesthesia (a mixture of 1-1.5% isoflurane and oxygen enriched air), the right femoral artery was cannulated percutaneously. An 8 Fr arterial sheath was introduced, and 100 mg/kg of lidocaine and 250 U/kg of heparin were administered intra-arterially. Additional heparin was administered during PTCA if needed, to maintain an activated coagulation time of > 300 seconds.

### Procedure

An 8 Fr right Amplatz guiding catheter and right Judkins guiding catheter were used for cannulation of the left and right coronary arteries, respectively. A standard balloon catheter (corresponding to a balloon/artery ratio of 1.1-1.3: 1) was advanced over a 0.014" floppy guide wire, and 3 successive 30-second inflations at 10 atm pressure were made with a 30 second interval between each inflation. PTCA was performed on all 3 coronary meries of each animal. For local delivery, the InfusaSleeve catheter (LocalMed Inc.) was used, which permits safe drug delivery with negligible additional injury (7). After balloon dilatation, each coronary artery of an animal was randomized to receive either 600 µg of 17-beta estradiol (in 5 ml), vehicle alone (5 ml), or PTCA only. The vehicle 2-hydroxypropyl-beta-cyclodextrin (HPCD), and 17-beta estradiol were obtained from Sigma Chem. Co. For local delivery with the InfusaSleeve catheter, a proximal driving pressure of 10 atm and support balloon pressure of 6 atm were utilized.

### Intracoronary infusion

All 9 animals underwent cardiac catheterization at the end of 4 weeks. After a baseline coronary angiogram, selective cannulation of the proximal portion of a coronary artery was performed with a single lumen balloon catheter (TotalCross, Schneider) for the administration of vasoactive agents. Acetylcholine (Ach) in increasing concentrations of 10⁻⁷ M, 10⁻⁶ M, 10⁻⁵ M, 10⁻⁴ M, was successively infused through the lumen port of the catheter.
Each dose was administered for a duration of 3 minutes at a constant rate of 1 ml/min using an infusion pump. Coronary angiography was performed at the end of each dose. After infusion of the highest concentration of Ach (10⁻⁴ M and angiography, 100 µg of nitroglycerin was administered via the lumen port of the catheter, and a coronary angiogram performed. The same protocol was repeated for the other 2 coronary arteries. Heart rate, blood pressure, and ECG were monitored continuously throughout the experiment.

### Quantitative coronary angiography

Coronary angiography was performed with a single plane imaging system (Electromed Intl). Images were obtained in predetermined views which best demonstrated the vessel segment of interest and without overlap of branches. Care was taken to maintain the same angulation during angiography of a segment throughout the procedure. Ionic contrast (MD-76, Mallinckrodt Medical Inc) was used throughout the experiment. Images were captured at a frame speed of 30 frames/sec, and stored digitally. A segment of contrast-filled guiding catheter was included in every frame, for the purpose of calibration. Calibration was performed using the known diameter of the contrast-filled guiding catheter as the reference segment, to avoid error due to magnification. Coronary artery diameter measurements were made using a validated computerized edge-detection system (8). The midpoint of the injured segment was used for calculation of coronary artery diameter. For each analysis, coronary artery diameter measurements were performed in 3 consecutive end-diastolic frames, and the results averaged.

Measurements were performed by an independent observer blinded to the treatment group of the vessels.

### Immunohistochemistry

The animals were euthanized at 4 weeks. Under general anesthesia as described above, exsanguination was performed with replacement by 1 I of 0.9 % NaCl solution. The heart was perfusion-fixed in vivo with 2 I of I0 % buffered formalin at 200 mm Hg pressure. The heart was then removed, and the coronary arteries were harvested immediately. From the injured segment (identified in relation to side branches), serial sections of 3-5 mm were made, and stored in 10 % buffered formalin solution. The sections were then treated with incremental concentrations of alcohol followed by treatment with xylene and paraffin. Slices of 6 µm thickness were prepared, and stained with Verhoeff's stain for assessment of tissue response to injury. For each injured segment, 2 slices demonstrating maximal neointima response were selected for immunohistochemistry, and the results obtained from analysis of the cross sections were averaged. The % of reendothelialization and, the % of endothelial nitric oxide synthase (eNOS) expression were calculated as follows: (the total length of the luminal surface staining positively / the perimeter of the lumen) x 100, respectively. Analysis was performed by an independent examiner with no knowledge of the treatment groups to which the sections belonged. For lectin immunohistochemistry, the 6 µm slices were first treated with hydrogen peroxide and methanol to block endogenous peroxide, incubated with the *Dolichos biflorus* agglutinin (Sigma Chemical Co.) followed by treatment with 3,3'-diaminobenzidine (Vector Laboratories) and, subsequently counter-stained with hematoxylin. For immunohistochemistry of eNOS expression, after blocking of endogenous peroxide and non-specific antibodies, the slices were treated serially with the primary mouse anti-eNOS antibody (Bio/Can Scientific), the secondary goat anti-mouse antibody (Vector Laboratories), incubated with avidin-biotin (Vector Laboratories), treated with 3,3'-diaminobenzidine (Vector Laboratories) and finally counter-stained with hematoxylin. For both immunohistochemical examinations, normal porcine carotid artery slices were used as positive controls; whereas slices obtained from the injured coronary arteries and stained only with hematoxylin were used as negative controls.

### Statistical analysis

Values are expressed as mean ± SD. Comparison of basal coronary artery diameter among the 3 groups was made using the one-way analysis of variance test. Comparisons between basal coronary artery diameter and coronary artery diameter following infusion of vasoactive agents were made with two-tailed Student's t-tests. The Kruskal-Wallis test was used for comparison of lectin and eNOS expression among the 3 treatment groups. Linear relationships between lectin expression and response to Ach, and between eNOS expression and response to Ach were analyzed with Pearson correlation coefficients. Values were considered to be statistically significant if p < 0.05.

### Results

There were no significant differences in basal coronary artery diameter (2.53 ± 0.6 mm for 17-beta estradiol, 2.79 ± 0.35 mm for PTCA only, and 2.77 ± 0.44 mm for vehicle groups respectively, p < 0.4) among the 3 treatment groups. The extent of morphologic tissue injury (9) among the groups was similar. No changes in heart rate, ECG, or blood pressure were noted during the local delivery or during intracoronary infusion of vasoactive agents.

### Response of PTCA only group to Ach

Compared to the basal coronary artery diameter, there were no significant changes in coronary artery diameter following intracoronary infusion of 10⁻⁷ M and 10⁻⁶ M concentrations of Ach (Table). At a concentration of 10⁻⁴ M a significant vasoconstrictive response was noted (p < 0.02). A marked vasoconstrictive response was observed at a concentration of 10⁻⁴ M (p < 0.0001) (Figure 3). The vasoconstriction was completely reversed upon administration of the endothelium-independent vasodilator nitroglycerin. Coronary diameter increased from 1.8 ± 0.48 mm after I0⁻⁴ M Ach, to 2.5 ± 0.28 mm following nitroglycerin (p < 0.01; p = 0.2 for post-nitroglycerin vs basal diameter).

### Response of vehicle treatment group to Ach

Compared to the basal coronary artery diameter, 10⁻⁷ M Ach did not change coronary artery diameter in the vehicle treatment group (Table 3). A trend towards significant vasoconstriction was noted with 10⁻⁶ M Ach (p = 0.06). Significant vasoconstriction was produced by 10⁻⁵ M (p < 0.02), and at 10⁻⁴ M (p < 0.001) Ach infusion respectively (Figure 3). Nitroglycerin completely reversed the vasoconstriction, returning the arteries to their basal diameter (from 1.89 ± 0.51 mm after 10⁻⁴ M Ach, to 2.69 ± 0.52 mm following nitroglycerin [p < 0.004; p = 0.7 for post-nitroglycerin vs basal diameter]).

### Response of 17-beta estradiol treated group to Ach

In the vessels treated with local delivery of 17-beta estradiol no significant vasoconstrictive response to Ach occurred at any concentration used (Table) (Figure 3). A mild and statistically nonsignificant increase in coronary artery diameter was observed following administration of nitroglycerin: from 2.28 ± 0.61 mm after 10⁻⁴ M Ach to 2.61 ± 0.48 mm after nitroglycerin (p = 0.4; p = 0.8 for post-nitroglycerin vs basal diameter).

### Immunohistochemistry

Immunohistochemical analyses were performed 4 weeks after PTCA on all 9 animals. Three arterial segments were lost/damaged during harvesting of the samples (2 of PTCA only group, and I of vehicle group). Significant differences were seen among the 3 treatment groups in the extent of reendothelialization, as assessed by immunohistochemical analysis with the lectin *Dolichos biflorus* agglutinin (Figure 4). Reendothelialization was noted to a greater extent in vessels treated with local delivery of 17-beta estradiol compared to the other 2 groups (90.6 ± 5.5 % for 17-beta estradiol 71 ± 6.8 % for PTCA only, and 72.8 ± 4.9 % for vehicle, p < 0.0005). Endothelial nitric oxide synthase expression was also higher in vessels treated with 17-beta estradiol (35.6 ± 11.8 % for 17-beta estradiol 9.4 ± 3.9 % for PTCA only, and 9.2 ± 4.0 % for vehicle, p < 0.0005) (Figure 5). No significant differences in immunohistochemical analyses were observed between vessels treated with vehicle or PTCA only.

We proceeded further to analyze whether a linear relationship between reendothelialization and the response to Ach could be demonstrated. A significant inverse correlation was noted between reendothelialization as assessed by immunohistochemistry with the lectin *Dolichos biflorus* agglutinin and the response to Ach (r = 0.48, p < 0.02) (Figure 6). An even stronger inverse linear correlation was observed between eNOS expression and the response to Ach (r = -0.58, p < 0.005).

### Conclusions

This study demonstrates for the first time that local delivery of 17-beta estradiol immediately following PTCA enhances subsequent reendothelialization and endothelial function at the site of injury. Besides its critical role in the regulation of vascular tone, the normal endothelium functions as an effective barrier between blood elements and underlying vascular smooth muscle cells. Endothelium-derived nitric oxide (NO) is a potent vasodilator, inhibits monocyte adherence and platelet aggregation and adhesion (10), vascular smooth muscle cell migration (11) and proliferation (12).

PTCA is associated with arterial injury and damage to the endothelium (3). Following arterial injury, varying rates of reendothelialization have been reported. Reendothelialization rates of 81 % (13), and even lower rates of < 50 % (14) following arterial injury have been observed. In a study of specimens of restenotic lesions obtained by atherectomy in humans, no endothelial cells could be demonstrated (15). In the present study, local treatment with 17-beta estradiol was followed by nearly complete reendothelialization (90.6 ± 5.5 %), which was significantly greater than that observed in the groups not treated with 17-beta estradiol. Estrogen receptors have been identified in human coronary artery and umbilical vein endothelial cells (16), and when bound to estrogen are capable of regulating protein synthesis by altering transcription rates (17). In cell culture assay of human umbilical vein endothelial cells, treatment with 17-beta estradiol markedly increased both cell migration and proliferation (18). Therapy with subcutaneously implanted 17-beta estradiol pellets significantly enhanced reendothelialization following arterial injury (6). The capacity of 17-beta estradiol to increase vascular endothelial growth factor synthesis (19) and the effort of 17-beta estradiol on basic fibroblast growth factor may be responsible for the enhanced reendothelialization. Vascular endothelial growth factor treatment is known to promote reendothelialization in vivo (20). In human umbilical vein and coronary artery endothelial cell culture experiments, treatment with 17-beta estradiol enhanced the release and phosphorylation of basic fibroblast growth factor (21,22). It has been shown that administration of basic fibroblast growth factor in vivo stimulates reendothelialization following arterial injury in rats (23). Another mechanism by which 17-beta estradiol could possibly influence extent of reendothelialization is by inhibition of apoptosis of injured endothelial cells: a 50 % decrease in apoptosis was seen with 17-beta estradiol treatment of human umbilical vein endothelial cells exposed to tumor necrosis factor-α (24). It is noteworthy that increased expression of tumor necrosis factors is known to occur following balloon injury (25).

Impaired endothelial function, as in atherosclerosis (26) or following experimental inhibition of NO (27), has been associated with a paradoxical constrictive response to Ach. This paradoxical response to Ach could be modified by treatment with estrogen. In humans, 17-beta estradiol administered intravenously (28) or by continuous intracoronary infusion (29), attenuated the vasoconstrictive response to Ach and also inhibited the Ach-induced increase in coronary resistance and decrease in coronary blood flow. The regulatory effect of 17-beta estradiol on eNOS that we observed may be responsible for the beneficial effects on endothelial function, as vascular response to Ach is closely related to eNOS expression (30,31 ). In support of this notion, a strong inverse linear relationship was seen between the vascular response to Ach and eNOS expression (Figure 4). The ability of estrogen to induce nitric oxide synthase was first identified during gestation in guinea pigs (32). Induction of eNOS function by 17-beta estradiol has been subsequently demonstrated to be accompanied by increased eNOS protein and mRNA expression (33,34). Increased circulating NO levels have been observed in postmenopausal women treated with 17-beta estradiol (35). Following arterial injury, the regenerated endothelium is often functionally abnormal (5). Abnormal vasomotion as a result of persistent endothelial dysfunction at the site of angioplasty has been demonstrated in patients undergoing PTCA, and is postulated to be responsible for the symptom of angina noted in patients with nonsignificant stenosis following PTCA (36). We have shown that functional abnormalities could be improved significantly by treatment with locally delivered 17-beta estradiol. A unifying hypothesis for the responses we observed is that eNOS downregulation following PTCA prevents the vasodilatory response to Ach mediated by endothelial NO production. By improving eNOS expression, 17-beta estradiol allows the vasodilatory response of Ach to counteract its direct vasoconstricting action, preventing Ach-induced vasoconstriction at the site of local injury. The vasodilatory response to nitroglycerin in Ach-constricted arteries post-PTCA is consistent with this concept, since exogenous nitroglycerin (which is a NO donor) simply provides a local NO-related dilation that the eNOS deficient angioplastied segment cannot provide for itself.

Both rapid non-genomic and genomic effects have been postulated to be involved in the influence of 17-beta estradiol on coronary vasculature (37,38). Although increased protein synthesis was not quantified in the present study, the enhanced eNOS expression and the response to Ach observed as late as 28 days following a single dose of 17-beta estradiol appears to be consistent with a genomic effect. This is the first study to suggest the existence of a genomic effect following local therapy with 17-beta estradiol in coronary circulation in vivo.

Gender differences in the endothelium-dependent vasodilation by 17-beta estradiol have been noted (39). In our study, a majority of animals were mates and a significant beneficial effect of 17-beta estradiol was noted in all the animals studied, irrespective of sex. Thus, local delivery of 17-beta estradiol appears to be effective in males as well as females. There is evidence to suggest that the simultaneous administration of progesterone reduces NO levels induced by 17-beta estradiol (35), this issue was, however, beyond the scope of the present study.

We conclude that a single dose of 17-beta estradiol delivered locally following balloon injury can significantly improve reendothelialization and enhance endothelial function at the injured site as late as 1 month following injury. Besides the beneficial vascular effects of improved endothelial function, this observation may be of particular importance following balloon angioplasty as improved endothelial function is known to be associated with decreased neointima formation in the injured area (20,40). This approach merits further study, with a view to potential clinical value in the prevention of vascular dysfunction and restenosis following PTCA.

### Formulations

The formulations may include estradiol or a derivative thereof and any pharmaceutically acceptable vehicle. Since estradiol is a lipophilic molecule, such vehicle would ideally include a solvent component. Such a solvent component includes molecules such as propylene glycol, ethanol, and detergents, for example Pluronics™. The formulations may take the form of a liquid, a suspension, a semi-solid or a thermoreversible composition which may form a layer over the endothelium. The formulations may further be included in or used as a coating for a device such as a stent, or be part of any similar device that can be left *in-situ* upon angioplasty or vascular surgery.

**Table 1:**

| Morphometric Analysis | | | | |
|---|---|---|---|---|
| Characteristics | 17-beta estradiol | PTCA only | Vehicle alone | p value* |
| Segments analyzed | 12 | 9 | 10 | NS |
| Artery size (mm) | 2.86 ± 0.35 | 2.94 ± 0.24 | 2.94 ± 0.41 | NS |
| Balloon/Artery ratio | 1.22 ± 0.09 | 1.2 ± 0.06 | 1.17 ± 0.11 | NS |
| EEL_{ref}/EELᵢₙⱼ† | 1.01 ± 0.16 | 1.31 ± 0.37 | 1.16 ± 0.28 | NS |
| Neointima area (mm²) | 0.4 ± 0.3 | 0.88 ± 0.61 | 1.14 ± 1.03 | < 0.05 |
| % neointima | 12.16 ± 8.89 | 23.02 ± 11.91 | 25.46 ± 14.96 | < 0.025 |
| Neointima/Media area | 0.59 ± 0.48 | 1.67 ± 1.29 | 1.75 ± 1.29 | < 0.01 |
| % stenosis | 15.67 ± 11.13 | 27.51 ± 13.17 | 30.34 ± 17.05 | < 0.025 |
| Restenotic index | 1.3 ± 0.5 | 2.4 ± 0.68 | 2.42 ± 0.71 | < 0.005 |
| Injury score | 1.64 ± 0.34 | 1.7 ± 0.43 | 1.77 ± 0.47 | NS |

| | | | | |
|---|---|---|---|---|
| * 17-beta estradiol vs other 2 groups; †EEL_{ref} = proximal reference segment external elastic lamina area, EELᵢₙⱼ = injured segment external elastic lamina area (averaged). | | | | |

**Table 2:**

| Response to 17-beta estradiol According to Sex of the Animal | | | |
|---|---|---|---|
| Characteristics | Male | Female | p value |
| Restenotic index | 1.2 ± 0.59 | 1.37 ± 0.45 | > 0.1 |
| Neointima area (mm²) | 0.51 ± 0.34 | 0.25 ± 0.15 | > 0.1 |
| Neointima/Media area | 0.78 ± 0.55 | 0.32 ± 0.16 | > 0.1 |
| % neointima | 14.93 ± 10.68 | 8.29 ± 3.72 | > 0.1 |
| % stenosis | 18.93 ± 13.39 | 11.09 ± 5.16 | > 0.1 |

**Table 3:**

| Response to Intracoronary Acetylcholine | | | |
|---|---|---|---|
| Ach* | Diameter-basal (mm) | Diameter-post Ach (mm) | p value |
| *PTCA group* | | | |
| 10⁻⁷M | 2.79 ± 0.35 | 2.65 ± 0.35 | 0.4 |
| 10⁻⁶M | 2.79 ± 0.35 | 2.54 ± 0.32 | 0.1 |
| 10⁻⁵M | 2.79 ± 0.35 | 2.3 ± 0.35 | 0.02 |
| 10⁻⁴M | 2.79 ± 0.35 | 1.8 ± 0.48 | 0.0001 |

| *Vehicle group* | | | |
|---|---|---|---|
| 10⁻⁷M | 2.77 ± 0.44 | 2.6 ± 0.41 | 0.4 |
| 10⁻⁶M | 2.77 ± 0.44 | 2.33 ± 0.5 | 0.06 |
| 10⁻⁵M | 2.77 ± 0.44 | 2.24 ± 0.47 | 0.02 |
| 10⁻⁴M | 2.77 ± 0.44 | 1.89 ± 0.51 | 0.001 |

| *17-beta estradiol group* | | | |
|---|---|---|---|
| 10⁻⁷M | 2.53 ± 0.6 | 2.46 ± 0.58 | 0.8 |
| 10⁻⁶M | 2.53 ± 0.6 | 2.38 ± 0.58 | 0.6 |
| 10⁻⁵M | 2.53 ± 0.6 | 2.36 ± 0.59 | 0.6 |
| 10⁻⁴M | 2.53 ± 0.6 | 2.28 ± 0.61 | 0.4 |

| | | | |
|---|---|---|---|
| * acetylcholine | | | |

### References cited in Example 1

¹ Dangas G, Fuster V. Management of restenosis after coronary intervention. Am Heart J 1996 ;132 :428-36.
² Post MJ, Borst C, Kuntz RE. The relative importance of arterial remodelling compared with intima hyperplasia in lumen narrowing after balloon angioplasty. Circulation 1994; 89: 2816-21.
³ Currier JW, Faxon DP. Restenosis after percutaneous transluminal coronary angioplasty: Have we been aiming at the wrong target? J Am Coll Cardiol 1995; 25: 516-20.
⁴ Teirstein PS, Massullo V, Jani S, Popma JJ, Mintz GS, Russo RJ, Schatz RA, Guarneri EM, Steuterman S, Morris NB, Leon MB, Tripuraneni P. Catheter-based radiotherapy to inhibit restenosis after coronary stenting. N Engl J Med 1997; 336: 1697-703.
⁵ King SBIII, Williams DO, Chougule P, Klein JL, Waksman R, Hilstead R, Macdonald J, Anderberg K, Crocker IR. Endovascular beta-radiation to reduce restenosis after coronary balloon angioplasty: results of the Beta Energy Restenosis Trial (BERT). Circulation 1998; 97: 2025-30.
⁶ Clowes AW, Reidy MA, Clowes MM. Kinetics of cellular proliferation after arterial injury: smooth muscle cell growth in the absence of endothelium. Lab Invest 1983; 49: 327-33.
⁷ Akishita M, Ouchi Y, Miyoshi H, Kozaki K, Inoue S, Ishikawa M, Eto M, Toba K, Orimo H. Estrogen inhibits cuff- induced intima thickening of rat femoral artery: effects on migration and proliferation of vascular smooth muscle cells. Atherosclerosis 1997; 130: 1-10.
⁸ Kolodgie FD, Jacob A, Wilson PS, Carlson GC, Farb A, Verma A, Virmani R. Estradiol attenuates directed migration of vascular smooth muscle cells in vitro. Am J Pathol 1996; 148: 969-76.
⁹ Dai - Do D, Espinosa E, Liu G, Rabelink TJ, Julmy F, Yang Z, Mahler F, Luscher TF. 17-beta estradiol inhibits proliferation and migration of human vascular smooth muscle cells: similar effects in cells from postmenopausal females and in males. Cardiovascular Research 1996; 32: 980-5.
¹⁰ Sullivan Jr TR, Karas RH, Aronovitz M, Faller GT, Ziar JP, Smith JJ, O'Donnell Jr TF, Mendelsohn ME. Estrogen inhibits the response - to - injury in a mouse carotid artery model. J Clin Invest 1995; 96: 2482-8.
¹¹ Chen SJ, Li H, Durand J, Oparil S, Chen YF. Estrogen reduces myointima proliferation after balloon injury of rat carotid artery. Circulation 1996; 93: 577-84.
¹² Moura A, Lam JYT, Hebert D, Kermode JR, Grant GW, Robitaille D, Klein EJ, Yock PG, Simpson JB, Kaplan AV. Intramural delivery of agent via a novel drug - delivery sleeve: histological and functional evaluation. Circulation 1995; 92: 2299-2305.
¹³ Varenne O, Pislaru S, Gillijns H, Pelt NV, Gerard RD, Zoldhelyi P, Van de Werf F, Collen D, Janssens S. Local adenovirus - mediated transfer of human endothelial nitric oxide synthase reduces luminal narrowing after coronary angioplasty in pigs. Circulation 1998; 98: 916-26.
¹⁴ Bonan R, Paiement P, Scortichini D, Cloutier MJ, Leung TK. Coronary restenosis: evaluation of a restenosis injury index in a swine model. Am Heart J 1993; 126: 1334-40.
¹⁵ Karas SP, Gravanis MB, Santoian EC, Robinson KA, Anderberg KA, King III SB. Coronary intima proliferation after balloon injury and stenting in swine: an animal model of restenosis. J Am Coll Cardiol 1992; 20: 467-74.
¹⁶ Mori T, Durand J, Chen YF, Thompson JA, Oparil S. Short term estrogen treatment prior to and following balloon injury of rat carotid artery effectively blunts the vascular injury response. J Am Coll Cardiol 1999; 33 (2 suppl A): 259A (abstract).
¹⁷ Foegh ML, Asotra S, Howell MH, Ramwell PW. Estradiol inhibition of arterial neointima hyperplasia after balloon injury. J Vasc Surg 1994; 19(4): 722-6.
¹⁸ Colburn P, Buonassis V. Estrogen - binding sites in endothelial cell cultures. Science 1978; 201: 817-9.
¹⁹ Venkov CD, Rankin AB, Vaughan DE. Identification of authentic estrogen receptor in cultured endothelial cells: a potential mechanism for steroid hormone regulation of endothelial function. Circulation 1996; 94: 727-33.
²⁰ Losordo DW, Kearney M, Kim EA, Jekanowski J, Isner JM. Variable expression of the estrogen receptor in normal and atherosclerotic coronary arteries of premenopausal women. Circulation 1994; 89: 1501-10.
²¹ Karas RH, Patterson BL, Mendelsohn ME. Human vascular smooth muscle cells contain functional estrogen receptor. Circulation 1994; 89: 1943-50.
²² Kuiper CiGMJ, Enmark E, Pelto - Huikko M, Nilsson S, Gustafsson JA. Cloning of a novel estrogen receptor expressed in rat prostrate and ovary. Proc Natl Acad Sci USA 1996; 93: 5925-5930.
²³ Mosselman S, Polman J, Dijkema R. ER/3: identification and characterization of a novel human estrogen receptor. FEBS Left 1996; 392: 49-53.
²⁴ lafrati MD, Karas RH, Aronovitz M, Kim S, Sullivan Jr TR, Lubahn DB, O'Donnell Jr TF, Korach KS, Mendelsohn ME. Estrogen inhibits the vascular injury response in estrogen receptor a-deficient mice.
²⁵ Hishikawa K, Nakaki T, Marumo T, Suzuki H, Kato R, Saruta T. Up - regulation of nitric oxide synthase by estradiol in human aortic endothelial cells. FEBS Lett 1995; 360: 291-3.
²⁶ Hayashi T, Yamada K, Esaki T, Kuzuya M, Satake S, Ishikawa T, Hidaka H, Iguchi A. Estrogen increases endothelial nitric oxide by a receptor - mediated system. Biochem Biophys Res Commun 1995; 214(3): 847-55.
²⁷ Rosselli M, Imthum B, Keller PJ, Jackson EK, Dubey RK. Circulating nitric oxide (nitrite/nitrate) levels in postmenopausal women substituted with 170-estradiol and norethisterone acetate: a two-year follow-up study. Hypertension 1995; 25(part 2): 848-53.
²⁸ Sarkar R, Meinberg EG, Stanley JC, Gordon D, Webb RC. Nitric oxide reversibly inhibits the migration of cultured vascular smooth muscle cells. Circ Res 1996; 78: 225-230.
²⁹ Cornwell TL, Arnold E, Boerth NJ, Lincoln TM. Inhibition of smooth muscle cell growth by nitric oxide and activation of cAMP-dependent protein kinase by cGMP. Am J Physiol 1994; 267: C1405-13.
³⁰ Speir E, Yu ZX, Ferrans VJ, Cannon III RO. Estrogen inhibits transcription factor and cell adhesion molecule activation in cytokine-stimulated human coronary smooth muscle cell via antioxidant effects. Circulation 1998; suppl I: I-220 (abstract).
³¹ Tanaka H, Sukhova GK, Swanson SJ, Clinton SK, Ganz P, Cybulsky MI, Libby P. Sustained activation of vascular cells and leucocytes in the rabbit aorta after balloon injury. Circulation 1993; 88: 1788-1803.
³² Yasukawa H, Imaizumi T, Matsuoka H, Nakashima A, Morimatsu M. Inhibition of intima hyperplasia after balloon injury by antibodies to intercellular adhesion molecule-1 and lymphocyte function - associated antigen-1. Circulation 1997; 95: 1515-22.
³³ Hyder SM, Stancel GM, Chiappetta C, Murthy L, Boettger-Tong HL, Makela S. Uterine expression of vascular endothelial growth factor is increased by estradiol and tamoxifen. Cancer Res 1996; 56(17): 3954-60.
³⁴ McLaren J, Prentice A, Charnock-Jones DS, Millican SA, Muller KH, Sharkey AM, Smith SK. Vascular endothelial growth factor is produced by peritoneal fluid macrophages in endometriosis and is regulated by ovarian steroids. J Clin Invest 1996; 98: 482-9.
³⁵ Asahara T, Bauters C, Pastore C, Kearney M, Rossow S, Bunting S, Ferrara N, Symes JF, Isner JM. Local delivery of vascular endothelial growth factor accelerates reendothelialization and attenuates intima hyperplasia in balloon-injured rat carotid artery. Circulation 1995; 91: 2793-2801.
³⁶ Mendelsohn ME, Karas RH. Estrogen and the blood vessel wall. Current Opinion in Cardiology 1994; 9: 619-26.
³⁷ Geary RL, Adams MR, Benjamin ME, Williams JK. Conjugated equine estrogens inhibit progression of atherosclerosis but have no effect an intima hyperplasia or arterial remodelling induced by balloon catheter injury in monkeys. J Am Coll Cardio) 1998; 31: 1158-64.
³⁸ Levine RL, Chen SJ, Durand J, Chen YF, Oparil S. Medroxyprogesterone attenuates estrogen-mediated inhibition of neointima formation after balloon injury of the rat carotid artery. Circulation 1996; 94: 2221-7.
³⁹ Oparil S, Levine RL, Chen SJ, Durand J, Chen YF. Sexually dimorphic response of the balloon-injured rat carotid artery to hormone treatment. Circulation 1997; 95: 1301-7.
⁴⁰ Lindner V, Kim SK, Karas RH, Kuiper GGJM, Gustafsson JA, Mendelsohn ME. Increased expression of estrogen receptor-f3 mRNA in male blood vessels after vascular injury. Circ Res 1998; 83: 224-9.
⁴¹ Brewster ME, Hora MS, Simpkins JW, Bodor N. Use of 2-hydroxypropyl-betacyclodextrin as a solubilizing and stabilizing excipient for protein drugs. Pharm Res 1991; 8(6): 792-5.
⁴² Frijlink HW, Visser J, Hefting NR, Oosting R, Meijer DKF, Lerk CF. The pharmacokinetics of beta-cyclodextrin and 2-hydroxypropyl-beta-cyclodextrin in the rat. Pharm Res 1990; 7(12): 1248-52.
⁴³ Kristinsson JK, Fridriksdottir H, Thorisdottir S, Sigurdardottir AM, Stefansson E, Loftsson T. Dexamethasone-cyclodextrin-polymer co-complexes in aqueous eye drops: aqueous humor pharmacokinetics in humans. Invest Ophthalmol Vis Sci 1996; 37: 1199-1203.
⁴⁴ Doenicke A, Roizen MF, Nebauer AE, Kugler A, Hoernecke R, Beger-Hintzen H. A comparison of two formulations for etomidate, 2-hydroxypropyl-beta-cyclodextrin (HPCD) and propylene glycol. Anesth Analg 1994; 79: 933-9.
⁴⁵ Hoon TJ, Dawood Y, Khan-Dawood FS, Ramos J, Batenhorst RL. Bioequivalence of a 17-beta estradiol hydroxypropyl-beta-cyclodextrin complex in postmenopausal women. J Clin Pharmacol 1993; 33: 1116-21.
⁴⁶ O'Keefe JH, Kim SC, Hall RR, Cochran VC, Lawhorn SL, McCallister BD. Estrogen replacement therapy after coronary angioplasty in women. J Am Coll Cardiol 1997; 29: 1-5.
⁴⁷ O'Brien JE, Peterson ED, Keeler GP, Berdan LG, Ohman EM, Faxon DP, Jacobs AK, Topol EJ, CaliffRM. Relation between estrogen replacement therapy and restenosis after percutaneous coronary interventions. J Am Coll Cardiol 1996; 28: 1111-8.

### References cited in Example 2

¹ Furchgott RF, Zawadzki JV. The obligatory role of endothelial cells in the relaxation of arterial smooth muscle by acetylcholine. Nature 1980; 288: 373-6.
² Rubanyi GM. The role of endothelium in cardiovascular homeostasis and diseases. J Cardiovasc Pharmacol 1993; 22(Suppl. 4): S1 -S 14.
³ Fischell TA, Derby G, Tse TM Stadius ML. Coronary artery vasoconstriction routinely occurs after percutaneous transluminal coronary angioplasty: a quantitative arteriographic analysis. Circulation 1988; 78: 1323-34.
⁴ Chesebro JH Lam JY, Badimon L, Fuster V. Restenosis after arterial angioplasty: a hemorrheologic response to injury. Am J Cardiol 1987; 60: 10B-16B.
⁵ Shimokawa H Aarhus LL, Vanhoutte PM. Porcine coronary arteries with regenerated endothelium have a reduced endothelium-dependent responsiveness to aggregating platelets and serotonin. Cire Res 1987; 61: 256-70.
⁶ Krasinski K, Spyridopoulos I, Asahara T, et al. Estradiol accelerates functional endothelial recovery after arterial injury. Circulation 1997; 1768-72.
⁷ Moura A, Lam JYT, Hebert D, et al. Intramural delivery of agent via a novel drug- delivery sleeve: histologic and functional examination. Circulation 1995; 92: 2299- 2305.
⁸ Mancini GBJ, Simon SB, McGillem MJ, et al. Automated quantitative coronary arteriography: morphologic and functional validation in vivo of a rapid digital angiographic method. Circulation 1987; 75(2): 452-60.
⁹ Karas SP, Gravanis MB, Santoian EC, et al. Coronary intima proliferation after balloon injury and stenting in swine: an animal model of restenosis. J Am Coll Cardiol 1992; 20: 467-74.
¹⁰ Cooke JP, Tsao PS. Cytoprotective effects of nitric oxide. Circulation 1993; 88(5): 2451-4.
¹¹ Sarkar R, Meinberg EG, Stanley JC, et al. Nitric oxide reversibly inhibits the migration of cultured vascular smooth muscle cells. Circ Res 1996; 78: 225-230.
¹² Cornwell TL, Arnold E, Boerth NJ, Lincoln TM. Inhibition of smooth muscle cell growth by nitric oxide and activation of cAMP-dependent protein kinase by cGMP. Am J Physiol 1994; 267: C1405-13.
¹³ Hayashi Y, Tomoike H Nagasawa K., et al. Functional and anatomical recovery of endothelium H1090.
¹⁴ Lindner V, Reidy MA Fingerie J. Regrowth of arterial endothelium: denudation with minimal trauma leads to complete endothelial cell growth. Lab Invest 1989; 61: 556- 63.
¹⁵ Bauriedel G, Windstetter U, DeMario Jr SJ, et al. Migratory activity of human smooth muscle cells cultivated from coronary and peripheral primary and restenotic lesions removed by percutaneous atherectomy. Circulation 1992; 85: 554-64.
¹⁶ Kim-Schulze S, McGowan KA, Hubchak SC, et al. Expression of an estrogen receptor by human coronary artery and umbilical vein endothelial cells. Circulation 1996; 94: 1402-7.
¹⁷ Venkov CD, Rankin AB, Vaughan DE. Identification of authentic estrogen receptor in cultured endothelial cells: a potential mechanism for steroid hormone regulation of endothelial function. Circulation 1996; 94: 727-33.
¹⁸ Morales DE, McGowan KA, Grant DS, et al. Estrogen promotes angiogenic activity in human umbilical vein endothelial cells in vitro and in a murine model. Circulation 1995; 91: 755-63.after denudation of coronary artery. Am J Physiol 1988; 254: H1081-
¹⁹ Hyder SM Stancel GM Chiappetta C, et al. Uterine expression of vascular endothelial growth factor is increased by estradiol and tamoxifen. Cancer Res 1996; 56(17):3964-60.
²⁰ Asahara T, Bauters C, Pastore C, et al. Local delivery of vascular endothelial growth factor accelerates reendothelialization and attenuates intima hyperplasia in balloon- injured rat carotid artery. Circulation 1995; 91: 2793-2801.
²¹ Kim-Schulze S, Lowe WL, Schnapper HW. Estrogen stimulates delayed mitogen-activated protein kinase activity in human endothehal cells via an autocrine loop that involves basic fibroblast growth factor. Circulation 1998; 98: 413-21.
²² Albuquerque ML, Akiyama SK, Schnaper HW. Basic fibroblast growth factor release by human coronary artery endothelial cells is enhanced by matrix proteins, 17-beta estradiol and a PKC signaling pathway. Exp Cell Res 1998; 245(1): 163-9.
²³ Lindner V, Majack RA, Reidy MA- Basic fibroblast growth factor stimulates endothelial regrowth and proliferation in denuded arteries. J Clin Invest 1990; 85: 2004-8.
²⁴ Spyridopoulos I Sullivan AB, Kearney M et al. Estrogen-receptor - mediated inhibition of human endothelial cell apoptosis: estradiol as a suraval factor. Circulation 1997; 95: 1505-14.
²⁵ Tanaka H Sukhova G, Schwartz D, Libby P. Proliferating arterial smooth muscle cells after balloon injury express TNF-α but not interleukin-1 or basic fibroblast growth factor. Arterioscier Thromb Vasc Biol 1996; 16: 12-18.
²⁶ Ludmer PL, Selwyn AP, Shook TL, et al. Paradoxical vasoconstriction induced by acetylcholine in atherosclerotic coronary arteries. N Eng J Med 1986; 315: 1046-5 1.
²⁷ Collins P, Burman J, Chung H Fox K. Hemoglobin inhibits endothelium-dependent relaxation to acetylcholine in human coronary arteries in vivo. Circulation 1993; 87: 80-5.
²⁸ Reis SE, Gloth ST, Blumenthal RS, et al. Ethinyl estradiol acutely attenuates abnormal coronary vasomotor responses to acetylcholine in postmenopausal women. Circulation 1994; 89: 52-60.
²⁹ Gilligan DM Quyyumi AA, Cannon III RO. Effects of physiological levels of estrogen on coronary vasomotor function in postmenopausal women. Circulation 1994; 89: 2545-51.
³⁰ Seo KK Yun HY, Kim H Kim SC. Involvement of endothelial nitric oxide synthase in the impaired endothelium-dependent relaxation of cavernous smooth muscle in hypercholesterolemic rabbit. J Androl 1999; 20(2): 298-306.
³¹ Kullo IJ, Mozes G, Schwartz RS, et al. Enhanced endothelium-dependent relaxations after gene transfer of recombinant endothelial nitric oxide synthase to rabbit carotid arteries. Hypertension 1997; 30(part 1): 314-20.
³² Weiner CP, Lizasoain I Baylis SA, et al. Induction of calcium-dependent nitric oxide syntheses by sex hormones. Proc Natl Acad Sci USA 1994; 91: 5212-16.
³³ Hishikawa K., Nakaki T, Marumo T, et al. Up-regulation of nitric oxide synthase by estradiol in human aortic endothelial cells. FEBS Letters 1995; 360: 291-3.
³⁴ MacRitchie AN, Jun SS, Chen Z, et al. Estrogen upregulates endothelial nitric oxide synthase gene expression in fetal pulmonary artery endothelium. Circ Res 1997; 81: 355-62.
³⁵ Rosselli M Imthum B, Keller PJ, et al. Circulating nitric oxide (nitrite/nitrate) level.% in postmenopausal women substituted with 17β-estadiol and norethisterone acetate: a two-year follow-up study. Hypertension 1995; 25(part 2): 848-53.
³⁶ Malekianpour M Doucet S, Lesperance J, et al. Abnormal coronary vasomotion and angina after successful coronary angioplasty. Circulation 1996; 94(suppl 1): I-560.
³⁷ Williams JK, Adams MR, Herrington DM, Clarkson TB. Short-term administration of estrogen and vascular responses of atherosclerotic coronary arteries. J Am Coll Cardio 1992; 20: 452-7.
³⁸ Wellman GC, Bonev AD, Nelson MT, Brayden JE. Gender differences in coronary artery diameter involve estrogen, nitric oxide, and Ca2+-dependent K+ channels. Circ Res 1996; 79: 1024-30.
³⁹ Kawano H, Motoyama T, Kugiyama K, et al. Gender differences in improvement of endothelium-dependent vasodilation after estrogen supplementation. J Am Coll Cardiol 1997; 30: 914-9.
⁴⁰ Chandrasekar B, Tanguay JF. Local delivery of 17-beta estradiol decreases neointima hyperplasia following coronary angioplasty in porcine model. (Submitted for publication).

## Claims

1. The use of 17-beta estradiol or a derivative thereof for the manufacture of a medicament or a device for improving reendothelization and vascular endothelial function in a patient, wherein the medicament or device is to be administered in-situ in the lumen of a blood vessel having suffered vascular injury, at the injured site.

2. The use as defined in claim 1, wherein 17-beta estradiol or a derivative thereof is to be administered in a dosage of 1 to 5000 µg/Kg of patient's body weight.

3. The use as defined in claim 1, wherein 17-beta estradiol or a derivative thereof is to be administered in a dosage of 10 to 50 µg/Kg of patient's body weight.

4. The use as defined in claim 1, wherein 17-beta estradiol or a derivative thereof is to be administered in a dosage of 10 to 30 µg/Kg of patient's body weight.

5. The use as defined in any one of claims 1 to 4, wherein the medicament or device further comprises a pharmaceutically acceptable carrier comprising hydroxypropyl-beta-cyclodextrin (HPCD).

6. The use as defined in claim 5, wherein 17-beta estradiol or a derivative thereof is solubilized in HPCD.

7. The use as defined in claim 4, where 17- beta estradiol or a derivative thereof is admixed with a carrier comprising at least 0.63 mg hydroxypropyl-beta-cyclodextrin per kilogram of patient's body weight.

8. The use as defined in any one of claims 1 to 7, which is for a single administration.

## Patentansprüche

1. Verwendung von 17β-Östradiol oder einem Derivat davon für die Herstellung eines Medikaments oder einer Vorrichtung zur Verbesserung der Reendothelisierung und vaskulären Endothelialfunktion bei einem Patienten, wobei das Medikament oder die Vorrichtung in situ in das Lumen eines Blutgefässes, das eine vaskuläre Verletzung erlitten hat, an die verletzte Stelle verabreicht wird.

2. Verwendung wie in Anspruch 1 definiert, wobei das 17β-Östradiol oder ein Derivat davon in einer Dosierung von 1 bis 5.000 µg/kg Körpergewicht des Patienten verabreicht wird.

3. Verwendung wie in Anspruch 1 definiert, wobei das 17β-Östradiol oder ein Derivat davon in einer Dosierung von 10 bis 50 µg/kg Körpergewicht des Patienten verabreicht wird.

4. Verwendung wie in Anspruch 1 definiert, wobei das 17β-Östradiol oder ein Derivat davon in einer Dosierung von 10 bis 30 µg/kg Körpergewicht des Patienten verabreicht wird.

5. Verwendung wie in einem der Ansprüche 1 bis 4 definiert, wobei das Medikament oder die Vorrichtung weiterhin einen pharmazeutisch akzeptablen Träger umfasst, umfassend Hydroxypropyl-β-cyclodextrin (HPCD).

6. Verwendung wie in Anspruch 5 definiert, wobei das 17β-Östradiol oder ein Derivat davon in HPCD löslich gemacht wird.

7. Verwendung wie in Anspruch 4 definiert, wobei das 17β-Östradiol oder ein Derivat davon mit einem Träger vermischt wird, umfassend mindestens 0,63 mg Hydroxypropyl-β-cyclodextrin pro kg Körpergewicht des Patienten.

8. Verwendung wie in einem der Ansprüche 1 bis 7 definiert, die für eine Einzelverabreichung gedacht ist.

## Revendications

1. Utilisation de 17-bêta oestradiol ou d'un de ses dérivés pour la fabrication d'un médicament ou d'un dispositif pour améliorer la réendothélialisation et la fonction endothéliale vasculaire chez un patient, dans laquelle le médicament ou le dispositif est à administrer in situ dans le lumen d'un vaisseau sanguin ayant souffert d'une blessure vasculaire, au site blessé.

2. Utilisation selon la revendication 1, dans laquelle le 17-bêta oestradiol ou l'un de ses dérivés est à administrer à un dosage de 1 à 5000 µg/kg de poids corporel du patient.

3. Utilisation selon la revendication 1, dans laquelle le 17-bêta oestradiol ou l'un de ses dérivés est à administrer à un dosage de 10 à 50 µg/kg de poids corporel du patient.

4. Utilisation selon la revendication 1, dans laquelle le 17-bêta oestradiol ou l'un de ses dérivés est à administrer à un dosage de 10 à 30 µg/kg de poids corporel du patient.

5. Utilisation selon l'une quelconque des revendications 1 à 4, dans laquelle le médicament ou le dispositif comprend en outre un support pharmaceutiquement acceptable comprenant l'hydroxypropyl-bêta-cyclodextrine (HPCD).

6. Utilisation selon la revendication 5, dans laquelle on solubilise le 17-bêta oestradiol ou l'un de ses dérivés dans l'HPCD.

7. Utilisation selon la revendication 4, dans laquelle on mélange le 17-bêta oestradiol ou l'un de ses dérivés avec un support comprenant au moins 0,63 mg d'hydroxypropyl-bêta-cyclodextrine par kilo de poids corporel du patient.

8. Utilisation selon l'une quelconque des revendications 1 à 7, qui est pour une administration unique.
